Europäisches Patentamt

European Patent Office

Office européen des brevets

(11) Numéro de publication: **0 218 770**
**A1**

## (12) DEMANDE DE BREVET EUROPEEN

(21) Numéro de dépôt: 85401979.1

(22) Date de dépôt: 11.10.85

(51) Int. Cl.4: **C12N 1/00** , C12N 1/38 , A01C 1/06

(43) Date de publication de la demande:
22.04.87 Bulletin 87/17

(84) Etats contractants désignés:
AT BE CH DE FR GB IT LI LU NL SE

(71) Demandeur: LABORATOIRES GOEMAR S.A.
Avenue du Général Patton Boîte Postale 55
F-35403 Saint-Malo(FR)

(72) Inventeur: Herve, René Ange
28 Chaussée du Sillon
F-35400 St. Malo(FR)
Inventeur: Percehais, Serge
55, Avenue George V
F-35800 Dinard Ille et Vilaine(FR)
Inventeur: Larher, François
27 Boulevard Jean Jaurès
F-54000 Nancy Meurthe et Moselle(FR)
Inventeur: Mourey-Bringuier, Anne
14, rue Gustave Charpentier
F-54500 Vandoeuvre Meurthe et Moselle(FR)

(74) Mandataire: Ranguis, Patrick Frédéric et al
Cabinet Beau de Loménie 55, rue
d'Amsterdam
F-75008 Paris(FR)

(54) **Utilisation de dérivés d'algues marines comme agents de culture, activateurs de croissance, et agents anti-stress, de micro organismes et champignons, et applications à l'enrobage des semences et aux engrais.**

(57) L'invention concerne l'application de filtrats de produits cryobroyés d'algues marines comme activateurs de croissance et anti-stress de micro organismes utiles eux-mêmes en agriculture ou en bioindustrie.

On citera notamment l'application au Rhizobium japonicum (soja) et au Rhizobium leguminosarum (petit pois).

Fig. 4

COURBES DE CROISSANCE DE RHIZOBIUM JAPONICUM G49 EN PRESENCE DE PROTOCHLORINE AMPICHI EN BODE À LA CONCENTRATION 5 g l⁻¹ ADDITIONNÉE DE DIFFÉRENTES SOURCES AZOTÉES A LA CONCENTRATION 10 mM
(o) MILIEU DE REFERENCE
(o) GLUTAMATE
(■) GLUTAMINE
(▲) ASPARAGINE
(▲) β ALANINE

## Utilisation de dérivés d'algues marines comme agents de culture, activateurs de croissance, et agents anti-stress, de micro organismes et champignons, et applications à l'enrobage des semences et aux engrais.

L'invention concerne des applications originales de certains produits dérivés d'algues marines.

On a décrit dans le brevet français n° 74 35162 un procédé de traitement d'algues dit par "cryobroyage".

Les algues sont traitées selon ce brevet de la manière suivante.

On récolte les algues, et on les lave en piscine afin de les débarrasser des animacules et du sable.

On leur fait ensuite subir une surgélation, notamment dans un surgélateur à plaques, à -10/-30°C, afin de permettre la conservation des éléments utiles.

On effectue ensuite en général une conservation en chambre froide en raison du fait que les récoltes sont saisonnières.

Selon ce brevet, on coupe puis congèle les algues et plantes à -20/-50°C, puis on les broie à cette température dans un broyeur à couteaux/broyeur à dents disposées en série. Une description détaillée de l'appareillage est donnée dans ce brevet. On envoie ensuite les algues et plantes (dimension moyenne 0,1-0,5 mm) vers un ou plusieurs broyeurs supplémentaires, à la température ambiante, par exemple broyeurs à trois cylindres (on atteint ainsi 100 à 50 $\mu$ ou même 10 $\mu$).

Les caractéristiques de ces appareils sont également décrites dans ledit brevet.

On obtient ainsi une "bouillie-mère" verte épaisse de pH voisin de 4,8 qui, grace au cryobroyage, contient la quasi-totalité des substances utiles et bénéfiques contenues dans les algues et plantes de départ (cf. Tableau I du brevet précité).

Les particules constitutives de cette "bouillie-mère" présentent une dimension de 6 à 20 $\mu$ environ. Cette "bouillie-mère" est aussi appelée "crème d'algues".

On peut faire passer cette "bouillie-mère" sur une décanteuse à grande vitesse qui donne deux produits, d'une part le gâteau que l'on dénomme "base d'algues" et d'autre part le jus de décantation que l'on dénomme "protoexoplasma d'algues".

Alternativement, à titre indicatif, cette "bouillie-mère" peut être également conditionnée en l'état.

Egalement à titre indicatif, il est possible d'effectuer une lyophilisation aussi bien de la crème d'algues que du protoexoplasma d'algues pour donner une forme sèche qui peut donner lieu par exemple à la fabrication de comprimés, etc.

Cette suite d'opérations est bien entendu valable pour chaque variété d'algues.

Dans le domaine de la préparation de ces différents extraits, on pourra se référer utilement au brevet français précité n° 74/35162 déposé le 18 octobre 1974.

On a également décrit dans la demande de brevet français n° 83-19142 déposée le 30 novembre 1983 un perfectionnement du procédé précédent, selon lequel on effectue la combinaison de quatre opérations :

1) cryobroyage tel que défini ci-dessus,

2) broyage moléculaire, par un appareillage spécifique,

3) turbodécantation (décantation à grande vitesse),

4) ultrafiltration sélective, qui permet d'obtenir un extrait nouveau d'algues et de plantes présentant certaines propriétés.

L'un des éléments essentiels du perfectionnement réside dans la combinaison des deux opérations 1) et 2).

En particulier, le "filtrat physiologique" obtenu après l'étape 4) est exempt de pavé cellulaire, de cellulose, de composants à longue chaîne moléculaire, et est directement assimilable (il contient micro-éléments, vitamines, micropeptides).

Une de ses caractéristiques est de ne pas contenir d'alcools résiduels provenant des étapes d'extraction.

Selon ce perfectionnement, les algues ou plantes fraîches sont coupées puis subissent un "cryobroyage" tel que décrit ci-dessus.

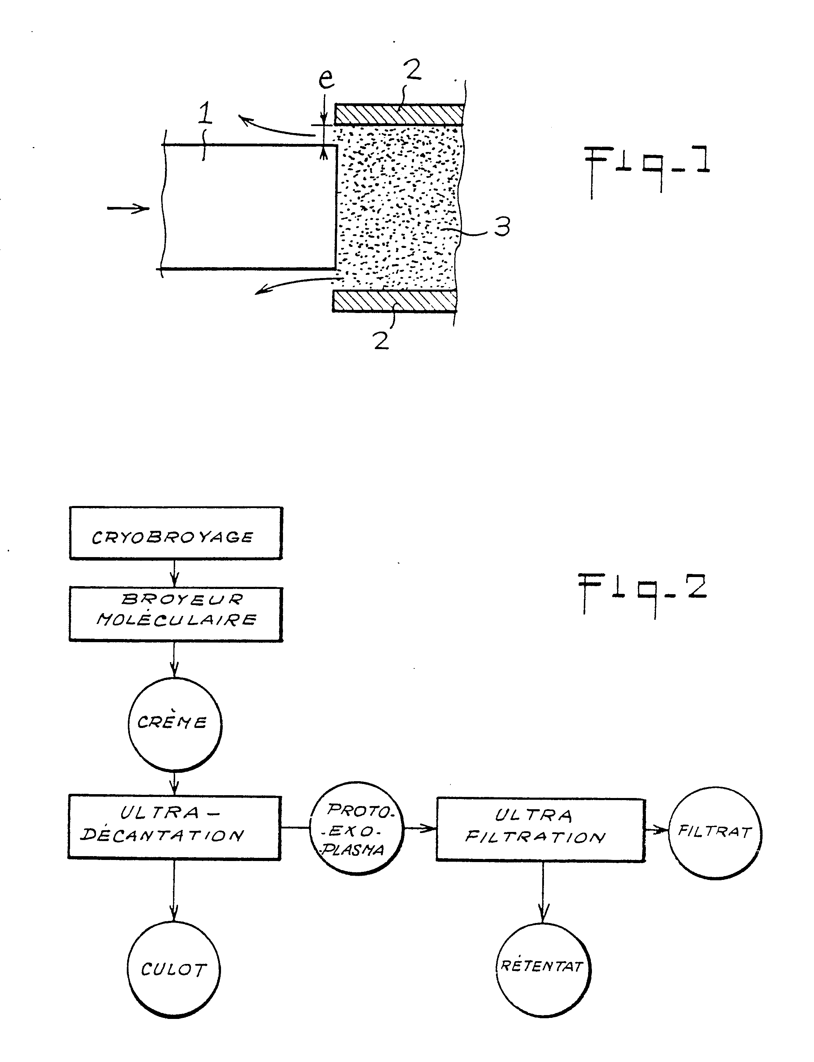

La bouillie est alors envoyée dans un broyeur moléculaire spécifique tel que représenté sur la figure 1 annexée.

Le broyeur spécifique nécessaire à l'obtention du produit selon l'invention consiste en un ensemble piston (1)/clapet (2) comportant entre le piston et le clapet un espace (e) extrêmement faible (de l'ordre de 0,035 mm sous 350 bars et à 400 litres/h).

Le principe est connu, le piston, par son mouvement de va-et-vient, chasse le produit ) traiter -(3) par l'espace (e) où ce produit est broyé.

On obtient selon ce procédé un produit broyé d'une manière nouvelle, ce qui libère d'autres principes que ceux libérés par les procédés connus, et de manière différente.

Ceci peut peut-être s'expliquer par l'effet de synergie que l'on observe, au niveau du broyage, entre les deux types de broyeurs.

Le procédé d'éclatement reposant sur une baisse brutale de la pression, qui peut devenir négative (effet de cavitation), entraîne, outre l'éclatement des cellules végétatives, l'éclatement des micro-organismes et en particulier des bactéries. On peut ainsi obtenir un produit stérile à condition toutefois que la pollution bactérienne initiale ne soit pas trop élevée.

La combinaison des deux broyeurs permet donc d'obtenir : . un produit plus actif du fait du plus grand pourcentage de cellules éclatées libérant une plus grande quantité de liquide intracellulaire ;

. une meilleure conservation des principes actifs, en particulier des propriétés enzymatiques conservées grâce au maintien d'une basse température au cours de toute la chaîne des opérations.

De plus, la conbinaison avec la technique de cryobroyage autorise le traitement des algues ou des plantes entières, pour la première fois.

La combinaison d'étapes selon l'invention ne réalise donc pas une extraction mais une "réduction" de la plante entière sous une forme microéclatée.

Enfin, la présence du broyeur "moléculaire" ou "cellulaire" dans la chaîne permet de mettre en oeuvre une ultra-filtration impossible à effectuer sans ce broyeur. Entre le broyage cellulaire et l'ultra-filtration, on effectue une ultra-décantation, elle aussi indispensable.

Le procédé selon l'invention consiste par conséquent en la combinaison des étapes suivantes :

-"cryobroyage",

-passage au broyeur moléculaire,

-ultra-décantation,

-ultra-filtration.

Pour certains produits, bien déterminés, on pourrait envisager de renoncer au broyeur moléculaire, mais pas dans le cas général.

L'étape 3) consiste en une turbodécantation avec pipettage du produit au centre du bol.

L'étape 4) d'ultra-filtration est effectuée sur des filtres de carbone comportant une couche d'oxyde de zirconium, membrane M4 [possédant un pouvoir de coupure inférieur à 200.000 (séparant les poids moléculaires > 200.000)].

Après l'étape 4), on peut conditionner en salle stérile ("salle blanche" ou "clean room") pour préserver la stérilité du produit qui est l'un des résultats essentiels de l'invention.

Cette chaîne d'opérations ainsi que les produits obtenus sont représentés sur la figure 2 annexée.

Dans le cas des algues, le "culot de centrifugation" contient principalement des débris cellulaires et des alginates.

Il peut être utilisé pour l'activation de composts et pour le traitement de certains effluents.

Le "rétentat" comprend des molécules de poids moléculaire supérieur à 200.000, en particulier des polysaccharides.

Il peut être utilisé pour la production de produits à usage externe mettant à profit les propriétés heparin-like des polysaccharides sulfurylés.

Le "filtrat physiologique" obtenu à l'étape 4) contient pratiquement tous les oligo-éléments, 80 % des polysaccharidés, les phytohormones et des vitamines et, d'une manière générale, toutes les molécules de poids inférieur à 200.000.

La présente invention concerne l'application des "filtrats physiologiques" dans un secteur technique tout à fait particulier qui est celui de la culture de micro organismes et champignons, notamment ceux utiles comme fixateurs de l'azote nécessaire à la croissance de certaines plantes.

On sait en effet que l'on a démontré assez récemment le grand intérêt de l'emploi de bactéries permettant l'assimilation de l'azote gazeux par les plantes (bactéries libres de la rhizosphère, ou bactéries susceptibles de contracter des relations symbiotiques avec les racines de légumineuses). Pour ce qui est des fixateurs d'azote symbiotiques (Rhizobiacées), il existe une spécificité d'hôte. Ainsi, Rhizobium japonicum est spécifique du soja, alors que Rhizobium leguminosarum est spécifique du pois (Pisum satinum).

Il est donc d'un intérêt capital de découvrir des milieux de culture, des agents favorisant la croissance et des agents "anti-stress" pour ces bactéries, de façon à les utiliser sous forme d'inoculums directement au champ ou indirectement sous forme stabilisée dans des enrobés de graines.

Par agents "anti-stress" on désigne des agents capables de protéger la bactérie utile contre, par exemple, les chocs thermiques ou hydriques ou salins qui pourraient la tuer au moment de son conditionnement ou inhiber sa croissance lors de l'infection racinaire.

L'invention propose de tels agents.

On a en effet découvert selon l'invention que la "crème d'algues", le "protoexoplasma d'algues" et surtout le "filtrat d'algues" décrits ci-dessus [pour de plus amples détails, on se reportera aux brevets précités] étaient très actifs dans les domaines ci-dessus.

On montrera en outre ci-dessous que, dans certaines conditions, il pourra être avantageux d'associer certains filtrats à des éléments tels que le bore, le molybdene, le calcium, et/ou à une source d'azote (acide aminé ou amide, asparagine, glutamine par exemple) et/ou à des protéines extraites de la cyanobactérie Spirulina.

On distinguera cinq champs d'application principaux des dérivés d'algues selon l'invention :

a) application comme milieu de culture et agent anti-stress pour des bactéries "symbiotiques" c'est-à-dire des bactéries qui coopèrent pour la fixation de l'azote moléculaire, au sein de nodosités racinaires des légumineuses.

b) mêmes applications pour les bactéries "non symbiotiques", c'est-à-dire dans un processus selon lequel le micro organisme fixe l'azote gazeux du sol, le réduit en ammoniac absorbé par la plante puisque ses racines se trouvent à proximité (fixation rhizosphérique).

c) mêmes applications pour les champignons ectomycorhizogènes.

L'enrobage des racines de conifères, par exemple, par ces champignons stabilisés, favorise la croissance.

d) mêmes applications pour les actynomycetes utiles pour la croissance, notamment, d'arbres forestiers tels que l'Aulne (fixation biologique de l'azote au sein d'actinorhizes).

e) culture de masse de levures pour la production de protéines.

Les processus que permet l'invention sont les suivants :

I. Culture de masse des micro organismes ou champignons sur le dérivé d'algue. Récupération des bactéries après culture, éventuellement avec le support de culture.

II A. Lyophilisation du produit ainsi récupéré ou

II B. Stabilisation du produit ainsi récupéré par addition de colloïdes (alginates, carraghenanes). On pourra notamment utiliser pour l'apport d'alginates la "crème d'algues" définie plus haut qui, outre des alginates, contient d'autres éléments utiles. On peut ensuite effectuer une précipitation par ajout de Ca $Cl_2$ à 17 % puis des lavages à l'eau pour éliminer l'excès de calcium et enfin des lavages à l'aide du "filtrat d'algues" brut ou additionné de solutés anti-stress pour apporter encore des éléments utiles.

On effectue ensuite une déshydratation. Pour cette déshydratation, on évitera les chocs thermiques et on préférera un simple séchage sous vide avec absorption de l'humidité par un piège de $CaCl_2$. Pour éviter le risque de fort "stress hydrique" durant la déshydratation, on effectue un apport de protéines solubles (notamment extrait de Spirulina)-

ou de solutés anti-stress qui, en outre, auront ultérieurement un effet bénéfique sur la croissance de la plante.

III. Le produit lyophilisé (II.A) ou déshydraté - (II.B) est ensuite soumis à un broyage pour obtenir une poudre fine. On utilisera par exemple un broyeur à billes.

IV. Conservation de ce produit pulvérulent de préférence :

-sous vide partiel

-à l'abri de la lumière

-au froid (∿ 4°C)

A titre de variante, il est avantageux d'effectuer un ajout d'un sucre (éventuellement apporté lui-même sous forme d'un dérivé d'algues) obtenu en V.

V. Les produits IV A ou IV B ainsi obtenus, que l'on peut encore associer aux éléments nécessaires à la croissance des plantes, sont alors :

-soit conservés à l'état de poudre,

-soit fixés sur la graine juste avant semis au moyen d'un liant constitué par la carboxylmethylcellulose, l'huile de soja ou une mousse de polyméthanol,

-soit fixés sur la graine de la même façon, graine qui est ensuite conservée dans cet état enrobé,

-soit utilisés pour "ensemencer" les racines des plantes.

Les exemples suivants illustrent l'invention sans toutefois en limiter la portée.

Exemple de préparation du "filtrat physiologique" d'algues :

Le filtrat physiologique (désigné ici par "GYFL" est un liquide protocellulaire d'algues fraîches sélectionnées (notamment Laminaria), et hautement purifié par le procédé physique sous froid - (cryobroyage, broyage moléculaire, turbodécantation, et ultra-filtration sélective) décrit plus haut.

-Cryobroyage et broyage moléculaire :

Les algues fraîches sont broyées sous froid (- 40°C) permettant d'obtenir une crème d'algues dont la finesse est inférieure à 20 $\mu$.

-Turbodécantation :

La crème d'algues est décantée à grande vitesse permettant d'éliminer la cellulose et les alginates insolubles.

-Ultra-filtration sélective :

Une filtration ultra-fine permet d'obtenir un liquide sans impuretés et dont les composants ont un poids moléculaire inférieur à 200.000.

On trouvera dans les tableaux I, II et III ci-après les analyses relatives à ce produit.

Exemple I :

CULTURE DE RHIZOBIUM JAPONICUM SUR "PROTOEXOPLASMA"

A -SUR PROTOEXOPLASMA BRUT

1°) Conditionnement des produits

Les solutions utilisées sont préparées à partir des crèmes brutes diluées à 20 g.l $^{-1}$ puis centrifugées à 4°C à 25000 tr.mn $^{-1}$ pendant 20 mn. Le surnageant recueilli, stérilisé est utilisé pour la culture en milieu liquide agité (28°C).

2°) Croissance sur les préparations (à différentes dilutions)

Le RHIZOBIUM JAPONICUM utilisé est une souche à hydrogénase (G 49).

-Le PROTOEXOPLASMA brut, aux concentrations de 1, 5 et 10 g.l $^{-1}$, inhibe la croissance du RHIZOBIUM. A la concentration de 0,1 g.l $^{-1}$, un taux de croissance significatif est observé (Figure 1).

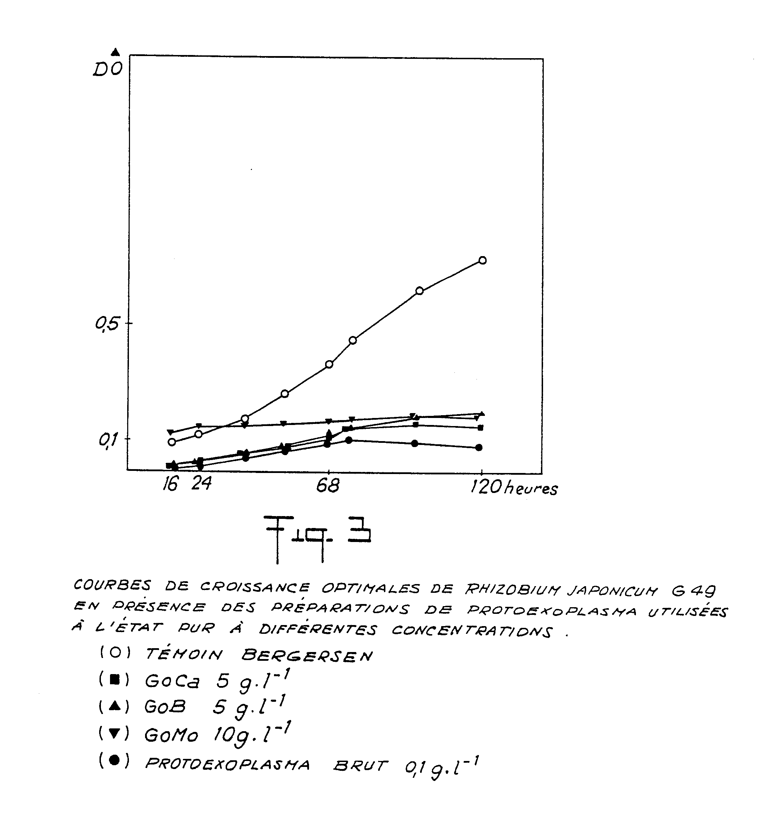

-Le PROTOEXOPLASMA enrichi en calcium - (GoCa) donne une croissance significative à 5 g.l $^{-1}$. Il en est de même pour les préparations enrichies en bore (GOB) (Figure 3).

-Le produit PROTOEXOPLASMA enrichi en MOLYBDENE (GoMo) permet une croissance à 10 g.l $^{-1}$ (Figure 3).

Dans tous les cas, les taux de croissance sont nettement inférieurs à ceux relevés sur le milieu de référence de Bergersen (Figure 3).

B -SUR PROTOEXOPLASMA ENRICHI EN UNE SOURCE D'AZOTE

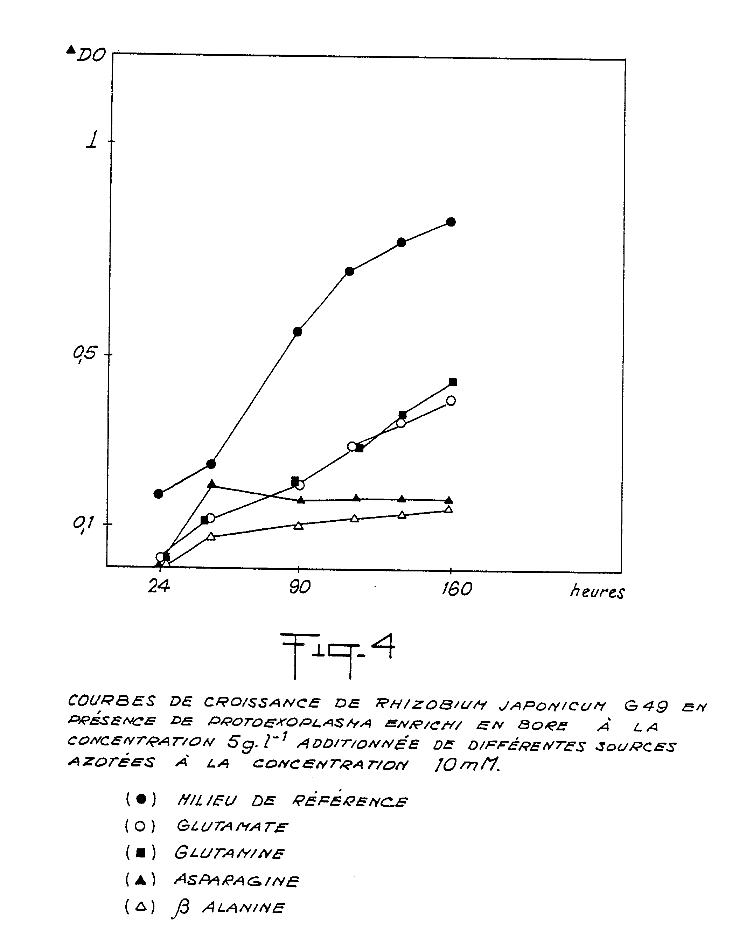

1°) PROTOEXOPLASMA enrichi en bore (Figure 4)

Des 4 composés mis en oeuvre, seuls deux sont utilisés rapidement et permettent une accélération de la croissance ; la glutamine et le glutamate.

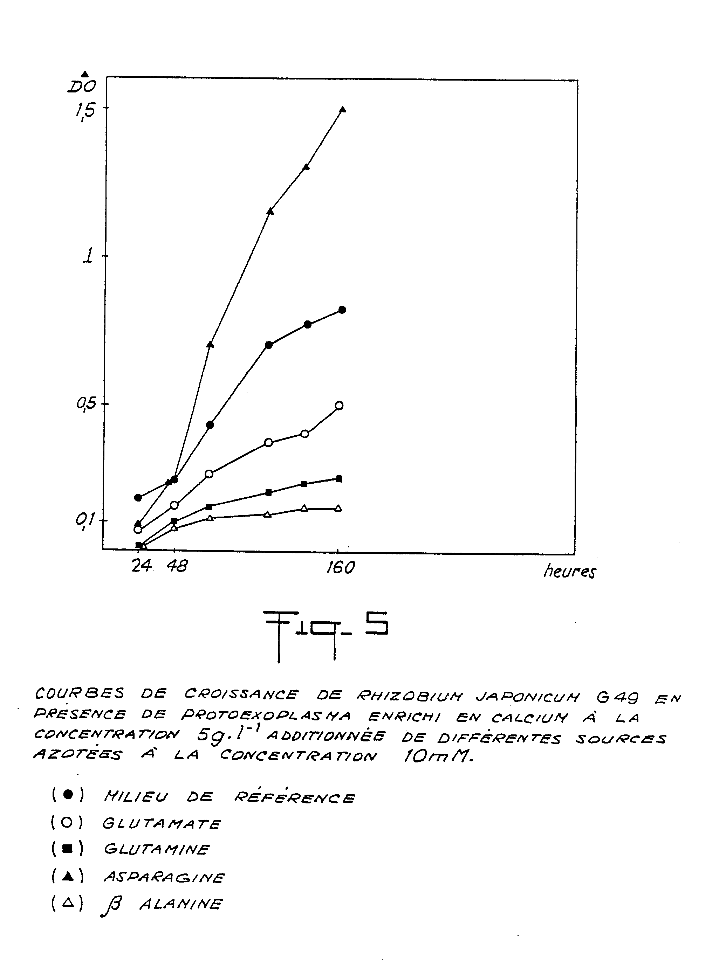

2°) PROTOEXOPLASMA enrichi en calcium - (Figure 5)

L'enrichissement de cette préparation en asparagine conduit à des croissances qui excèdent nettement celles obtenues sur le milieu de référence : le glutamate et la glutamine sont nettement moins bien utilisés.

Exemple 2 :

CULTURE DE DIFFERENTES SOUCHES DE RHIZOBIUM SUR LE FILTRAT PHYSIOLOGIQUE DE LAMINAIRE "GYFL"

A -CROISSANCE DE DIFFERENTES SOUCHES DE RHIZOBIUM JAPONICUM

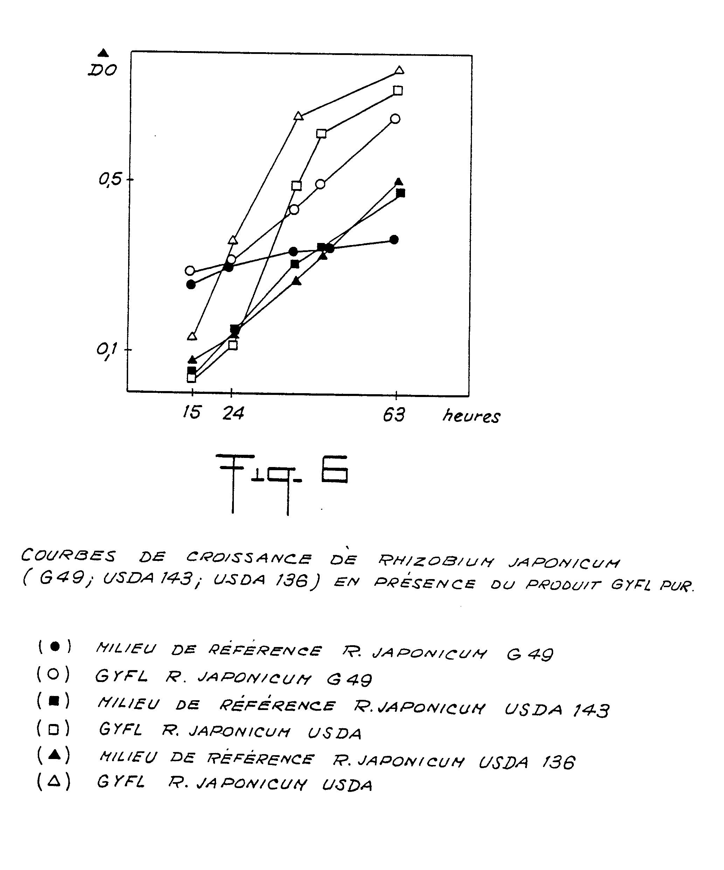

Les souches G 49, USDA 143 et USDA 136 sont retenues pour cette expérience (Figure 6). Le produit GYFL est stérilisé et utilisé à l'état pur sans aucune dilution préalable. Le produit GYFL assure aux trois souches de R. JAPONICUM une croissance nettement supérieure à celle observée sur le milieu de référence (en 90 h, les taux de croissance sont sensiblement multipliés par 2). Des essais ont également été réalisés sur le produit GYFL dilué 10, 50 et 100 fois (voir Tableau IV). Les taux de croissance sont nettement plus faibles qu'en présence du produit non dilué.

B -CROISSANCE DE QUELQUES AUTRES ESPECES DE RHIZOBIUM SUR GYFL NON DILUE

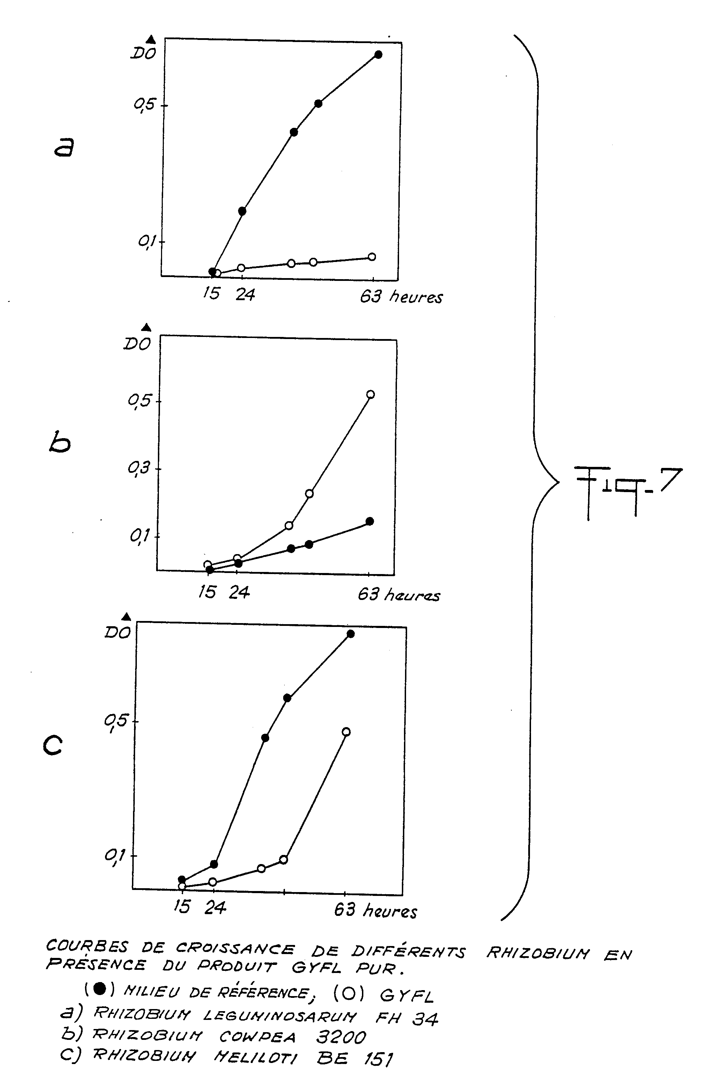

1°) Cas de R. LEGUMINOSARUM, R. COWPEA et R. MELILOTI (Figure 7)

Pour la première espèce (Figure 7), la croissance sur GYFL est très lente ; pour la seconde - (Figure 7), elle est nettement plus élevée que sur le milieu de référence X 3,5) ; pour la dernière - (Figure 7), après un temps de latence accru, on constate également une croissance satisfaisante.

2°) Cas de R. LUPINI

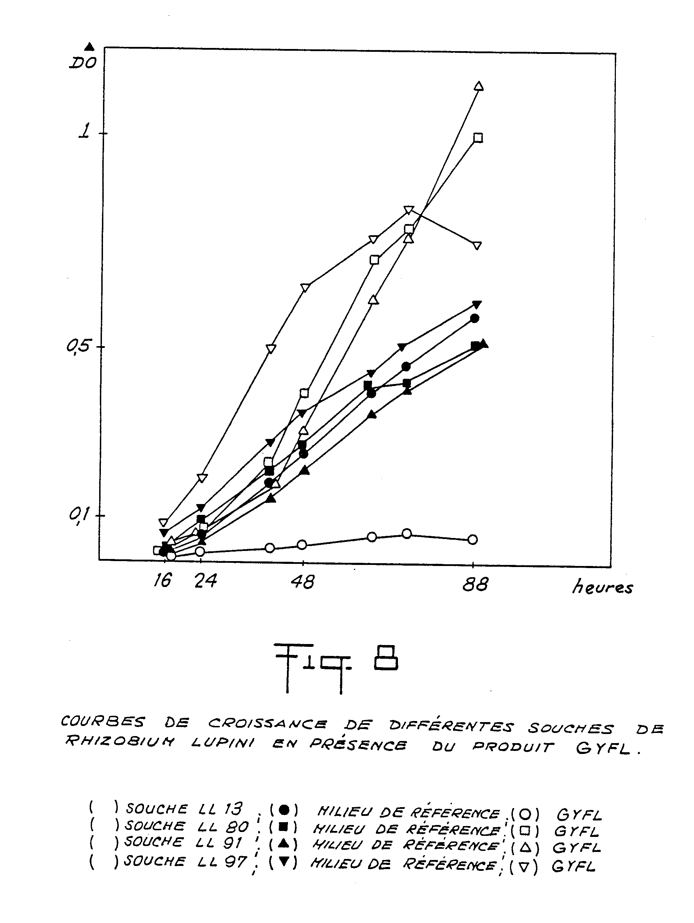

4 souches de cette bactérie sont soumises à l'expérimentation (Figure 8). Parmi les 4 souches étudiées, 3 se révèlent capables de croître plus rapidement sur GYFL que sur le milieu optimal de Bergersen. Il s'agit de LL 80, LL 91, LL 97. Par contre, la souche LL 13 ne présente qu'une croissance très ralentie.

En conclusion, le produit GYFL, non dilué constitue un milieu de culture idéal pour un certain nombre de bactéries symbiotiques fixatrices d'azote moléculaire.

Les exemples donnés ci-dessus permettront à l'homme du métier de sélectionner les espèces et les souches sur lesquelles le GYFL présente un effet bénéfique.

Par ailleurs, le produit PROTOEXOPLASMA - (micropulvérisat d'Ascophyllum nodosum) enrichi ou non en éléments tels que B, Mo, Ca, ne trouvera des applications que dans la mesure où il subira un enrichissement en une source azotée (acide aminé ou amide) à une concentration de l'ordre de 10 mM.

Les souches précitées sont accessibles sur demande, aux fins d'études et de recherches, au sens de l'article 14 bis de la loi française sur les brevets du 2-1-68 modifiée le 13-7-78, et de son décret d'application n° 81.865 auprès de l'INRA, Laboratoire de microbiologie des sols, B V 1540 - 21034 DIJON (France).

Leurs références sont les suivantes :

| | |
|---|---|
| RHIZOBIUM LUPINI | LL 13 |
| | LL 80 |
| | LL 91 |
| | LL 97 |
| LEGUMINOSARUM | FH 34 |
| COWPEA | 3200 |
| MELILOTI | BE 151 |
| JAPONICUM | G 49 |
| | USDA 143 |
| | USDA 136 |

## TABLEAU 1

### RESULTATS D'ANALYSES

Les résultats sont exprimés en pourcentage de produit humide

| ECHANTILLONS | CENDRES | SUBSTANCES ORGANIQUES | | |
|---|---|---|---|---|
| | | PROTEINES | LIPIDES | GLUCIDES* |
| GYFL | 0,312 % | 0,12 % | 0,02 % | 0,756 % |

* exprimés en "équivalent glucose"

TABLEAU II

GYFL

| | Méthode | Minéralisation | Précision % |
|---|---|---|---|
| SODIUM | Spectrométrie de flamme (a) | 1 | ± 5 |
| POTASSIUM | " " " (a) | 1 | 4 |
| LITHIUM | " " " | 1 | 4 |
| CALCIUM | Absorption atomique (flamme) - AFNOR V18-108 | 1 | 4 |
| MAGNESIUM | " " " - (b) | 1 | 4 |
| STRONTIUM | Spectrométrie de flamme | 1 | 4 |
| CHROME | Colorimétrie (Diphényl Carbazide) | 2 | / |
| FER | Absorption atomique (flamme) - (c) | 2 | ± 5 |
| MANGANESE | " " " - (c) | 2 | 4 |
| CUIVRE | " " (four) - (c) | 2 | ± 10 |
| COBALT | " " " | 2 | / |
| NICKEL | " " " | 2 | ± 10 |
| ZINC | Absorption atomique (flamme) - (c) | 2 | ± 5 |
| ETAIN | " " (four) | 2 | ± 10 |
| PLOMB | " " " | 2 | " |
| CADMIUM | " " " | 2 | " |
| MOLYBDENE | Colorimétrie (thiocyanate) | 2 | ± 5 |
| ANTIMOINE | Colorimétrie (Rhodamine) | 2 | / |
| TITANE | " (acide chromotropique) | 2 | ± 10 |
| CHLORE | Volumétrie au nitrate d'argent - (a) | 3 | ± 3 |
| PHOSPHORE | Colorimétrie à l'heptamolybdate | 4 | ± 5 |

0 218 770

TABLEAU II (suite)

GYFL

| | Méthode | Minéralisation | Précision % |
|---|---|---|---|
| AZOTE | Kjeldalh (volumétrie) | 4 | ± 5 |
| ARSENIC | Colorimétrie (cf AFNOR T 90201) | 2 | ± 10 |
| BORE | Colorimétrie (Curcumin) AFNOR T 90201 | 3 | ± 3 |
| IODE | " (méthode aux sels cériques) | 3 | ± 3 |
| BROME | " (phénolsulfonephtaléine) | 3 | ± 5 |
| SOUFRE | Népholométrie (sous forme SO4) | 1 | ± 5 |
| FLUOR | Colorimétrie (méthode au zirconium) AFNOR T 90004 | 3 | ± 5 |
| MERCURE | Absorption atomique sous flamme AFNOR T 90113 | 5 | ± 10 |
| SELENIUM | Polarographie | 6 | ± 10 |

Observations : 1 : Calcination et reprise des cendres par HCl
2 : Minéralisation humide H2SO4 + HNO3 + HClO4
3 : Calcination en milieu alcalin - reprise des cendres par eau
4 : Minéralisation humide (H2SO4 + H2O2)
5 : Minéralisation humide (H2SO4 + HNO3 + KMNO)
6 : Attaque à l'acide bromhydrique

Légende : (a) : 1e directive C.E.E. Aliments des animaux
(b) : 4e directive C.E.E.
(c) : 8e directive C.E.E.
(d) : 3e directive C.E.E.

0 218 770

TABLEAU III

RESULTATS DE L'ANALYSE

| | GYFL |
|---|---|
| SODIUM (Na) mg/kg | 816 |
| POTASSIUM (K) " | 850 |
| LITHIUM (Li) µg/kg | ≼15 |
| CALCIUM (Ca) mg/kg | 150 |
| MAGNESIUM (Mg) " | 118 |
| STRONTIUM (Sr) " | 1,700 |
| CHROME (Cr) " | ≺0,1 |
| FER (Fe) " | 2,9 |
| MANGANESE (Mn) " | 0,34 |
| CUIVRE (Cu) " | 0,31 |
| COBALT (Co) µg/kg) | ≺50 |
| NICKEL (Ni) " | 50 |
| MOLYBDENE (Mo) " | ≺20 |
| ZINC (Zn) " | 2,0 |
| ETAIN (Sn) mg/kg | 17 |
| PLOMB (Pb) " | ≺0,1 |
| CADMIUM (Cd) " | 0,042 |
| ANTIMOINE (Sb) " | ≺0,01 |
| CHLORE (Cl) " | 973 |
| PHOSPHORE (P) " | 176 |
| AZOTE (N) " | 188 |
| ARSENIC (As) " | 3,0 |
| BORE (B) " | 1,41 |
| IODE (I) " | 1,8 |
| BROME (Br) " | 16 |
| SOUFRE (S) " | 222 |
| TITANE (Ti) " | <1 |
| FLUOR (F) " | 8,4 |
| MERCURE (Hg) " | ≺0,05 |
| SELENIUM (Se) " | |

TABLEAU IV- Taux de croissance de *Rhizobium japonicum* au bout de 90 heures, sur milieu de référence Bergersen et sur GYFL à différentes concentrations.

| | Milieux de croissance | | | | |
|---|---|---|---|---|---|
| | Bergersen | GYFL pur | GYFL dilué 10 fois | GYFL dilué 50 fois | GYFL dilué 100 fois |
| D.O. (*) | 0,56 | 1,17 | 0,16 | 0,04 | 0,03 |
| Pourcentage de croissance | 100 % | 209 % | 28 % | 7 % | 5 % |

(*) Densité Optique

## Revendications

1/ -Utilisation de dérivés d'algues marines comme agents de culture, activateurs de croissance et agents anti-stress de microorganismes et champignons, caractérisée en ce que ces dérivés résultent

. du traitement d'algues ou plantes entières par la combinaison des étapes suivantes :

a) cryobroyage (congélation des algues ou plantes coupées à -20/ -50°C, puis broyage à cette température dans un broyeur à couteaux ou à dents, puis dans un ou plusieurs broyeurs à la température ambiante) donnant une bouillie de dimensions de particules100 à 50 ou 10 $\mu$ ;

b) broyage moléculaire

c) décantation à grande vitesse

d) ultra-filtration et récupération du filtrat liquide stérile ainsi obtenu.

. et sont éventuellement enrichis en bore, molybdène, calcium et analogues

2/- Utilisation selon la revendication 1, caractérisée en ce que l'algue appartient à la famille Laminaria.

3/- Utilisation selon la revendication 1 ou 2 pour la culture de microorganismes et champignons utiles comme fixateurs de l'azote nécessaire à la croissance des plantes.

4/- Utilisation selon la revendication 3, caractérisée en ce que la culture est celle de Rhizobium Japonicum.

5/- Dérivés d'algues marines qui résultent du traitement d'algues ou plantes entières par la combinaison des étapes suivantes :

a) cryobroyage (congélation des algues ou plantes coupées à -20/ -50°C, puis broyage à cette température dans un broyeur à couteaux ou à dents, puis dans un ou plusieurs broyeurs à la température ambiante) donnant une bouillie de dimension de particules 100 à 50 ou 10 $\mu$ ;

b) broyage moléculaire

c) décantation à grande vitesse

d) ultra-filtration et récupération du filtrat liquide stérile ainsi obtenu,

et sont éventuellement enrichis en bore, molybdène, calcium et analogues, caractérisés en ce qu'ils sont sous une forme appropriée pour la fixation sur des graines par un liant tel que carboxyméthylcellulose, huile de soja ou mousse de polyméthanol, ou l'ensemencement de racines de plantes.

6/ Graines caractérisées en ce qu'elles sont enrobées par au moins un dérivé d'algues selon la revendication 5.

e

1

2

3

Fig-1

Fig-2

CRYOBROYAGE

BROYEUR
MOLÉCULAIRE

CRÈME

ULTRA-
DÉCANTATION

PROTO-
EXO-
PLASMA

ULTRA
FILTRATION

FILTRAT

CULOT

RÉTENTAT

## Fig. 3

COURBES DE CROISSANCE OPTIMALES DE RHIZOBIUM JAPONICUM G 49
EN PRÉSENCE DES PRÉPARATIONS DE PROTOEXOPLASMA UTILISÉES
À L'ÉTAT PUR À DIFFÉRENTES CONCENTRATIONS.

(O) TÉMOIN BERGERSEN

(■) GoCa 5 g.$l^{-1}$

(▲) GoB 5 g.$l^{-1}$

(▼) GoMo 10g.$l^{-1}$

(●) PROTOEXOPLASMA BRUT 0,1 g.$l^{-1}$

**Fig. 4**

COURBES DE CROISSANCE DE RHIZOBIUM JAPONICUM G49 EN PRÉSENCE DE PROTOEXOPLASMA ENRICHI EN BORE À LA CONCENTRATION $5g.l^{-1}$ ADDITIONNÉE DE DIFFÉRENTES SOURCES AZOTÉES À LA CONCENTRATION 10mM.

(●) MILIEU DE RÉFÉRENCE

(○) GLUTAMATE

(■) GLUTAMINE

(▲) ASPARAGINE

(△) β ALANINE

Fig. 5

COURBES DE CROISSANCE DE RHIZOBIUM JAPONICUM G49 EN PRÉSENCE DE PROTOEXOPLASMA ENRICHI EN CALCIUM À LA CONCENTRATION 5g. $l^{-1}$ ADDITIONNÉE DE DIFFÉRENTES SOURCES AZOTÉES À LA CONCENTRATION 10mM.

( ● ) MILIEU DE RÉFÉRENCE

( O ) GLUTAMATE

( ■ ) GLUTAMINE

( ▲ ) ASPARAGINE

( △ ) β ALANINE

Fig. 6

COURBES DE CROISSANCE DE RHIZOBIUM JAPONICUM
( G49; USDA 143; USDA 136) EN PRÉSENCE DU PRODUIT GYFL PUR.

( ● )  MILIEU DE RÉFÉRENCE R. JAPONICUM  G 49
( ○ )  GYFL R. JAPONICUM G 49
( ■ )  MILIEU DE RÉFÉRENCE R. JAPONICUM  USDA 143
( □ )  GYFL R. JAPONICUM USDA
( ▲ )  MILIEU DE RÉFÉRENCE R. JAPONICUM USDA 136
( △ )  GYFL R. JAPONICUM USDA

a

b

c

Fig-7

COURBES DE CROISSANCE DE DIFFÉRENTS RHIZOBIUM EN
PRÉSENCE DU PRODUIT GYFL PUR.

(●) MILIEU DE RÉFÉRENCE; (O) GYFL
a) RHIZOBIUM LEGUMINOSARUM FH 34
b) RHIZOBIUM COWPEA 3200
c) RHIZOBIUM MELILOTI BE 151

Fig. 8

COURBES DE CROISSANCE DE DIFFÉRENTES SOUCHES DE
RHIZOBIUM LUPINI EN PRÉSENCE DU PRODUIT GYFL.

( ) SOUCHE LL 13 ; (●) MILIEU DE RÉFÉRENCE ;(O) GYFL
( ) SOUCHE LL 80 ; (■) MILIEU DE RÉFÉRENCE ;(□) GYFL
( ) SOUCHE LL 91 ; (▲) MILIEU DE RÉFÉRENCE ;(△) GYFL
( ) SOUCHE LL 97 ; (▼) MILIEU DE RÉFÉRENCE ;(▽) GYFL

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,Y | WO-A-8 402 652   (LABORATOIRES GOEMAR S.A.)<br>* En entier * | 1 | C 12 N    1/00<br>C 12 N    1/38<br>A 01 C    1/06 |
| | --- | | |
| Y | FR-A-2 091 427  (CESKOSLOVENSKA AKADEMIE VED. INSTITUTION D'ETAT)<br>* En entier * | .1 | |
| | --- | | |
| Y | US-A-3 123 538  (M. SHIROTA et al.)<br>* En entier * | 1 | |
| | --- | | |
| Y | CHEMICAL ABSTRACTS, vol. 73, no. 21, 23 novembre 1970, page 251, no. 108241a, Columbus, Ohio, US; & JP - A - 70 20 953 (CHLORELLA INDUSTRY CO., LTD.) 16-07-1970<br>* Résumé en entier * | 1 | DOMAINES TECHNIQUES RECHERCHES (Int. Cl.4) |
| | --- | | |
| A | US-A-3 889 418  (K.E. PORTER et al.) | | C 12 N<br>C 12 P<br>A 01 C<br>A 23 L<br>A 01 G |
| | --- | | |
| A | US-A-3 820 281  (E.R. BIGLER et al.) | | |
| | --- | | |
| A | FR-A-2 469 861  (ANVAR) | | |
| | ---                        -/- | | |

Le présent rapport de recherche a été établi pour toutes les revendications

| Lieu de la recherche<br>LA HAYE | Date d'achèvement de la recherche<br>11-06-1986 | Examinateur<br>DESCAMPS J.A. |
|---|---|---|

| | **DOCUMENTS CONSIDERES COMME PERTINENTS** | | Page 2 |
|---|---|---|---|
| Catégorie | Citation du document avec indication, en cas de besoin, des parties pertinentes | Revendication concernée | CLASSEMENT DE LA DEMANDE (Int. Cl.4) |
| E | FR-A-2 569 720 (S.A. LABORATOIRES GOEMAR)<br><br>--- | 1-6 | |
| D,A | FR-A-2 287 943 (R. HERVE et al.)<br><br>----- | | |
| | | | **DOMAINES TECHNIQUES RECHERCHES (Int. Cl.4)** |

Le présent rapport de recherche a été établi pour toutes les revendications

| Lieu de la recherche<br>LA HAYE | Date d'achèvement de la recherche<br>11-06-1986 | Examinateur<br>DESCAMPS J.A. |
|---|---|---|